# EUROPÄISCHE PATENTSCHRIFT

(11) **EP 2 185 662 B1**
(45) Veröffentlichungstag und Bekanntmachung des Hinweises auf die Patenterteilung: **22.12.2010**
(21) Anmeldenummer: 08787515.9
(22) Anmeldetag: 27.08.2008
(51) Int. Cl.: C09D 7/00, C09D 7/12

(54) **PHOTOAKTIVES TIO2 IN BESCHICHTUNGSMATERIALIEN**
PHOTOACTIVE TIO2 IN COATING MATERIALS
TIO2 PHOTOACTIF DANS DES MATÉRIAUX DE REVÊTEMENT

(30) Priorität: 28.08.2007 EP 07115107
(43) Veröffentlichungstag der Anmeldung: 19.05.2010
(73) Patentinhaber: BASF SE, 67056 Ludwigshafen (DE)
(72) Erfinder: SEEBER, Alexandra, 68165 Mannheim (DE); SCHINDLER, Götz-Peter, 67071 Ludwigshafen (DE); FREITAG, Katrin, 67065 Ludwigshafen (DE); JAHNS, Ekkehard, 69469 Weinheim (DE); RAFFAELE ADDAMO, Antonino, 67063 Ludwigshafen (DE); KLEINE JAEGER, Frank, 67098 Bad Dürkheim (DE); KLINGLER, Dirk, 68163 Mannheim (DE)
(86) Internationale Anmeldenummer: PCT/EP2008/061220
(87) Internationale Veröffentlichungsnummer: WO 2009/027432

(56) Entgegenhaltungen:
- WO-A-99/01766
- DATABASE WPI Week 200764 Thomson Scientific, London, GB; AN 2007-689318 XP002501462 & WO 2007/069596 A (ASAHI KASEI CHEM CORP) 21. Juni 2007 (2007-06-21)
- DATABASE WPI Week 200515 Thomson Scientific, London, GB; AN 2005-135623 XP002501463 & JP 2005 028575 A (DOKURITSU GYOSEI HOJIN BUSSHITSU ZAIRYO) 3. Februar 2005 (2005-02-03)
- DATABASE WPI Week 200665 Thomson Scientific, London, GB; AN 2006-624086 XP002501464 & JP 2006 233343 A (NIPPON SODA CO) 7. September 2006 (2006-09-07) in der Anmeldung erwähnt

## Beschreibung

Die vorliegende Erfindung betrifft ein Beschichtungsmaterial, umfassend wenigstens ein Bindemittel und wenigstens ein photokatalytisch aktives Teilchen, ein Verfahren zur Herstellung dieses Beschichtungsmaterials und die Verwendung dieses Beschichtungsmaterials.

Beschichtungsmaterialien, welche photokatalytisch aktive Teilchen enthalten, sind aus dem Stand der Technik bereits bekannt.

JP 11 181339 A offenbart ein hydrophiles Beschichtungsmittel für Metalle, Polymere, Beton, umfassend photokatalytisch aktive Titandioxid-Partikel. Die Titandioxid-Partikel gemäß JP 11 181339 A weisen einen Partikeldurchmesser von 1 bis 100 nm auf, enthalten Partikel aus SnO₂ und sind mit Silica und/oder einem Silikon beschichtet. Die Schichtdicke dieser Beschichtung aus Silica und/oder Silikon wird nicht offenbart. Das Beschichtungsmaterial vermeidet das Verschmutzen einer Fläche, die mit diesem Material beschichtet ist. Des Weiteren hat eine so behandelte Oberfläche selbstreinigende Eigenschaften, wenn Regen auf diese auftrifft. Daher sind die Beschichtungsmaterialien gemäß JP 11 181339 A zur Beschichtung von Oberflächen von Gebäuden, Fensterrahmen und anderen Gütern geeignet.

JP 2006-18 14 90 offenbart eine Beschichtungsmethode zur Beschichtung von Materialien. Dazu wird ein Photokatalysator, bestehend aus Titandioxid, Zirkoniumdioxid, Zinkoxid, Zinnoxid, Ceroxid, Antimonoxid, Indiumoxid dotiert mit Zinn, Zinnoxid dotiert mit Antimon mit einem nicht-wässrigen Lösungsmittel auf die zu schützenden Oberflächen aufgebracht. In einer bevorzugten Ausführungsform ist ein Photokatalysator bestehend aus Titandioxid mit Keramiken wie Apatit, Silica, aktiviertem Kohlenstoff, aktiviertem Alumina oder porösem Glas beschichtet, um den Photokatalysator auf der zu behandelnden Fläche zu befestigen. Das Verfahren gemäß diesem Dokument ist durch die Notwendigkeit des Aufbringens von wenigstens zwei Schichten kompliziert.

JP 2006-233343 offenbart eine flüssige Photokatalysator-Zusammensetzung zur Beschichtung von Textilien, welche Photokatalysator-Partikel mit voreingestelltem Partikel-Durchmesser und einer Beschichtung enthält. Die Größe dieser Photokatalysator-Partikel beträgt 0,5 bis 10 µm. In einer bevorzugten Ausführungsform besteht der Kern des Photokatalysator-Partikels aus Titandioxid, und die Hülle besteht aus Silica. Eine Dicke bzw. die Porosität der Hülle des photokatalytisch aktiven Partikels werden nicht offenbart.

JP 11228873 A offenbart eine Farbzusammensetzung umfassend mit porösem Silica beschichtete Titandioxid-Partikel, welche photokatalytische Eigenschaften aufweisen. Des Weiteren enthält die Zusammensetzung Titandioxid als Pigment und ein organisches Harz als Bindemittel. In JP 11228873 A wird nicht offenbart, welche Partikeldurchmesser die photokatalytisch aktiven Teilchen aufweisen, oder welche Schichtdicke die Silica-Schicht aufweist.

WO 2005/100459 A1 offenbart ein Beschichtungsmaterial mit einem Bindemittel und mindestens einem Teilchen mit einer Größe und/oder Oberflächenrauhigkeit von 100 µm oder weniger aufweisendem Füllstoff sowie einem katalytisch wirksamen Mittel, wobei das Bindemittel zumindest teilweise durch die photokatalytische Wirkung abgebaut wird und eine mikrostrukturierte, selbstreinigende Oberfläche entsteht. Als photokatalytisch wirksames Mittel wird gemäß WO 2005/100459 ein Oxid von Titan, Zink, Eisen, Mangan, Molybdän und/oder Wolfram eingesetzt, welches mindestens ein Additiv, ausgewählt aus C, N, S und/oder aus einem Übergangsmetalloxid und/oder -halogenid enthält. WO 2005/100459 A1 offenbart nicht, welche Partikelgröße die photokatalytisch aktiven Teilchen aufweisen.

EP 1 956 041 A1 offenbart wässrige organisch-anorganische Hybridzusammensetzungen enthaltend ein Metalloxid (A) mit einem Partikeldurchmesser von 1 bis 400 nm und einem Polymerpartikel (B) mit einem Partikeldurchmesser von 10 bis 800 nm. Das Metalloxid kann ausgewählt sein aus Siliciumdioxid, Aluminiumoxid, Antimonoxid, Titandioxid, Indiumoxid, Zinnoxid und anderen. Diese Photokatalysatoren können auch mit Siliciumoxid beschichtet sein. Das in der Zusammensetzung gemäß diesem Dokument vorliegende Polymer enthält zwingend Silan-Gruppen enthaltende Monomere, Vinylamid-enthaltende Monomere und gegebenenfalls weitere vinylische Monomere. Geeignete Lösungsmittel sind Wasser, gegebenenfalls in Kombination mit organischen Lösungsmitteln, wie beispielsweise Alkohole.

JP 2005 028575 A offenbart ultradünne poröse Oxidfilme, welche hergestellt werden durch abwechselndes Laminieren eines geschichteten Oxids im Nanometerbereich und Aluminiumhydroxiden auf einer Substrafischicht. Des Weiteren werden auch Kern-Schale-Partikel, die einen porösen ultradünnen Oxidfilm als Beschichtung auf einer Polymerkugel aufweisen, offenbart.

WO 99/01766 A offenbart Metallpartikel umfassend einen Metallkern und eine Silanenthaltende Schale, wobei der Kern ein Metall umfasst ausgewählt, aus der Gruppe bestehend aus Gold, Silber, Platin, Palladium, Rhodium, Ruthenium, Osmium, Iridium und Mischungen davon und die Schale einen Mercaptosilan-Rest, gebunden an diesen Metallkern, enthält. Des Weiteren offenbart dieses Dokument auch ein Material umfassend ein Matrixmaterial oder ein Trägermaterial, wobei darin die beschriebenen Metallpartikel eingebettet sein können, wobei das Matrixmaterial oder das Trägermaterial ausgewählt ist aus der Gruppe bestehend aus Polymeren, porösen Gläsern, gefärbten Gläsern, porösem Titandioxid, Zeolithen, Silica und anderen.

Die im Stand der Technik beschriebenen Beschichtungsmaterialien enthalten photokatalytisch aktive Partikel, welche den Nachteil aufweisen, dass nicht nur die auf dem Beschichtungsmaterial bei der Benutzung vorhandenen Verunreinigungen photokatalytisch abgebaut werden, was erwünscht ist, sondern auch das Bindemittel, welches in dem Beschichtungsmaterial vorhanden ist, ebenfalls photokatalytisch abgebaut wird. Dies ist nicht erwünscht und die im Stand der Technik beschriebene photokatalytische Zerstörung der organischen Matrices erniedrigt die Photoaktivität der erzeugten Oberfläche. Sowohl die Photoaktivität als auch die Haltbarkeit bzw. Widerstandsfähigkeit der Beschichtungsmaterialien gemäß dem Stand der Technik ist dadurch nur unzureichend.

Aufgabe der vorliegenden Erfindung ist es, ein Beschichtungsmaterial bereitzustellen, welches sich dadurch auszeichnet, dass auf dem Beschichtungsmaterial vorhandene Verschmutzungen durch das Beschichtungsmaterial abgebaut und entfernt werden oder dass superhydrophile Oberflächen erzeugt werden, und dass gleichzeitig das Beschichtungsmaterial eine hohe Widerstandsfähigkeit gegenüber Licht und Witterungseinflüssen aufweist.

Diese Aufgaben werden gelöst durch ein Beschichtungsmaterial, umfassend
(A) wenigstens ein Bindemittel als Komponente (A) und
(B) wenigstens ein photokatalytisch aktives Teilchen, umfassend einen nichtporösen Kern enthaltend wenigstens ein Metall- oder Halbmetalloxid mit einem Durchmesser von 0,1 nm bis 1 µm und wenigstens eine den Kern zumindest teilweise umgebende poröse Hülle enthaltend wenigstens ein weiteres Metall- oder Halbmetalloxid mit einer mittleren Schichtdicke von 0,1 bis 10 nm als Komponente (B).

Im Rahmen der vorliegenden Erfindung bedeutet Beschichtungsmaterial eine Mischung von Stoffen, die dazu geeignet ist, auf verschiedene Materialien aufgebracht zu werden und eine entsprechende, feste Beschichtung auszubilden. Übliche Beschichtungsmaterialien können neben den genannten Komponenten weitere Komponenten enthalten, beispielsweise Lösungsmittel, Tenside, Farbstoffe, Füllstoffe, Pigmentverteiler, Entschäumer und Polymere. Materialien, die mit Beschichtungsmitteln beschichtet werden können, können für Außen- oder Innenanwendungen vorgesehen sein, aus Metall, anorganischen oder organischen Materialien bestehen.

Die beiden zwingend vorliegenden Komponenten und optional vorliegende Komponenten des erfindungsgemäßen Beschichtungsmaterials werden im Folgenden näher erläutert.

### Komponente (A):

Als Komponente (A) enthält das erfindungsgemäße Beschichtungsmaterial wenigstens ein Bindemittel. In einer bevorzugten Ausführungsform ist das als Komponente (A) eingesetzte Bindemittel ausgewählt aus der Gruppe bestehend aus auf Wasser und/oder organischen Lösungsmitteln basierenden Kunststoffdispersionen auf Basis von nichtpolymerischen Vernetzungssystemen, Acrylaten, Caprolactam, Vinylcaprolactam, N-Vinylformamid, Acrylestern, Styrol/Acrylestern und Vinylacetaten, Polyurethanen, Epoxidharzen, Alkydharzen und Mischungen davon. Als Komponente (A) geeignete nicht-polymerische Vernetzungssystemen sind multifunktionale Methylolderivative von Harnstoff und substitutierten Harnstoffen, Melamin, Dimethylolharnstoff oder N,N'-Bis(hydroxymethyl)hamstoff, Methylolderivative von Melamin, die aus bis zu sechs Methylolgruppen pro Molekul bestehen, bevorzugt die, in den die Methylolgruppen teilweise oder völlig in Methoxymethylgruppen umgewandelt sind, 1,3-Bis(hydroxymethyl)tetrahydro-2-(1H)-pyrimidinon, Triazone wie Hydroxyethyltriazon, 5-Hydroxy-1,3-bis(hydroxymethyl)-hexahydro-s-triazin-2-on, Urone wie Tetrahydro-3,5-bis(hydroxymethyl)-4H-1,3,5-oxadiazin-4-on, Methylolderivative von Carbamaten, bevorzugt Methylcarbamat und Methoxyethylcarbamat, Methylolderivative von Dihydroxyethylenhamstoff, Dimethyloldihydroxyethylenharnstoffe (DMDHEU), Dimethyldihydroxyethylenhamstoffe, 4,5-Dihydroxy-1,3-dimethyl-2-imidazolidinon, Polycarboxysäuren wie Zitronensäure und Butantetracarboxysäure. Als Komponente (A) geeignete Polyacrylate im Sinne der vorliegenden Erfindung sind beispielsweise erhältlich durch Copolymerisation von mindestens einem (Meth)acrylsäure-C1-C1a-Alkylester, beispielsweise Acrylsäuremethylester, Acrylsäureethylester, Acrylsäure-n-Butylester, Methacrylsäure-n-butylester, Acrylsäure-2-ethylhexylester, mit mindestens einem weiteren Comonomer, beispielsweise einem weiteren (Meth)acrylsäure-C1-C10-Alkylester, (Meth)acrylsäure, (Meth)acrylamid, N-Methylol(meth)acrylamid, Glycidyl(meth)acrylat oder einer vinylaromatischen Verbindung wie beispielsweise Styrol. Als Komponente (A) geeignete vorzugsweise anionische Polyurethane im Sinne der vorliegenden Erfindung sind beispielsweise erhältlich durch Umsetzung von einem oder mehreren aromatischen oder vorzugsweise aliphatischen oder cycloaliphatischen Diisocyanat mit einem oder mehreren Polyesterdiolen und vorzugsweise einer oder mehreren Hydroxycarbonsauren, z. B Hydroxyessigsaure, oder vorzugsweise Dihydroxycarbonsäuren, beispielsweise 1,1-Dimethylolpropionsäure, 1,1-Dimethylolbuttersäure oder 1,1-Dimethylolethansäure. Als Komponente (A) besonders geeignete Ethylen-(Meth)acrylsaure-Copolymere sind beispielsweise durch Copolymerisation von Ethylen, (Meth)acrylsäure und gegebenenfalls mindestens einem weiteren Comonomer wie beispielsweise (Meth)acrylsäure-C1-C10-Alkylester, Maleinsäureanhydrid, Isobuten oder Vinylacetat erhältlich, vorzugsweise durch Copolymerisation bei Temperaturen im Bereich von 190 bis 350°C und Drücken im Bereich von 1500 bis 3500, bevorzugt 2000 bis 2500 bar. Als Komponente (A) besonders geeignete Ethylen-(Meth)acrylsäure-Copolymere können beispielsweise bis zu 90 Gew.-% Ethylen einpolymerisiert enthälten und eine Schmelzeviskosität im Bereich von 60 mm²/s bis 10.000 mm²/s, bevorzugt 100 mm²/s bis 5.000 mm²/s aufweisen, gemessen bei 120°C. Als Komponente (A) besonders geeignete Ethylen-(Meth)acrylsäure-Copolymere konnen beispielsweise bis zu 90 Gew.-% Ethylen einpolymerisiert enthalten und eine Schmelzemassefliessrate (MFR) im Bereich von 1 bis 50 g/10 min, bevorzugt 5 bis 20 g/10 min, besonders bevorzugt 7 bis 15 g/10 min aufweisen, gemessen bei 160°C und einer Belastung von 325 g nach EN ISO 1133. Als Komponente (A) besonders geeignete Copolymere von mindestens einem Vinylaromaten mit mindestens einem konjugierten Dien und gegebenenfalls weiteren Comonomeren, beispielsweise Styrol-Butadien-Bindemittel, enthalten mindestens eine ethylenisch ungesättigte Carbonsäure oder Dicarbonsäure oder ein geeignetes Derivat, beispielsweise das entsprechende Anhydrid, einpolymerisiert. Besonders geeignete Vinylaromaten sind para-Methylstyrol, α-Methylstyrol und insbesondere Styrol. Besonders geeignete konjugierte Diene sind Isopren, Chloropren und insbesondere 1,3-Butadien. Als besonders geeignete ethylenisch ungesättigte Carbonsäuren oder Dicarbonsäuren oder geeignete Derivate davon seien (Meth)acrylsäure, Maleinsäure, Itaconsäure, Maleinsäureanhydrid bzw. Itaconsäureanhydrid beispielhaft genannt. Des Weiteren kann Kaliwasserglas, insbesondere für Silikatfarben, und weitere übliche und dem Fachmann bekannte Bindemittel für Außen-, Innenfarben, Beschichtungsmittel für industrielle Anwendungen, technische Apparate und Spezialbeschichtungen, eingesetzt werden.

Komponente (A) liegt im Allgemeinen in einer Menge von 5 bis 99,9 Gew.-% vor. Wird das erfindungsgemäße Beschichtungsmaterial für Außenanwendungen eingesetzt, so liegt Komponente (A) bevorzugt in einer Menge von 5 bis 99,9 Gew.-%, besonders bevorzugt 5 bis 98,5 Ges.-% vor. Wird das erfindungsgemäße Beschichtungsmaterial für Innenanwendungen eingesetzt, so liegt Komponente (A) bevorzugt in einer Menge von 5 bis 99,5 Gew.-%, besonders bevorzugt 5 bis 98 Gew.-% vor.

### Komponente (B):

Als Komponente (B) umfasst das erfindungsgemäße Beschichtungsmaterial wenigstens ein oben beschriebenes photokatalytisch aktives Teilchen.

Komponente (B) liegt in dem erfindungsgemäßen Beschichtungsmaterial im Allgemeinen in einer Menge von 0,1 bis 10 Gew.-% vor, Wird das erfindungsgemäße Beschichtungsmaterial für Außenanwendungen eingesetzt, so liegt Komponente (B) bevorzugt in einer Menge von 0,1 bis 8 Gew.-%, besonders bevorzugt 0,5 bis 5 Gew.-%, vor. Wird das erfindungsgemäße Beschichtungsmaterial für Innenanwendungen eingesetzt, so liegt Komponente (B) bevorzugt in einer Menge von 0,5 bis 10 Gew.-%, besonders bevorzugt 2 bis 8 Gew.-%, vor.

Der nicht poröse Kern des photokatalytisch aktiven Teilchens enthält wenigstens ein Metall- oder Halbmetalloxid, und die wenigstens eine poröse Hülle des Nanopartikels enthält wenigstens ein weiteres Metall- oder Halbmetalloxid.

Geeignete Metalle oder Halbmetalle, deren Oxide im nicht porösen Kern des als Komponente (B) vorliegenden wenigstens einen photokatalytisch aktiven Teilchens vorliegen, sind im Allgemeinen ausgewählt aus der Gruppe bestehend aus Elementen der Gruppen I bis XV des Periodensystems der Elemente (nach IUPAC), Lanthanoiden. Actinoiden und Mischungen davon, besonders bevorzugt aus der Gruppe bestehend aus V, Ti, Zr, Ce, Mo, Bi, Zn, Mn, Si, Ba, Au, Ag, Pd, Pt, Ru, Rh, La und Mischungen davon.

Ein besonders bevorzugtes Metall- oder Halbmetalloxid, welches im nicht porösen Kern von Komponente (B) vorliegt, ist TiO₂, welches im wesentlichen in der Anatas-Modifikation vorliegt, d.h. bevorzugt zu mehr als 50%, besonders bevorzugt zu 60 bis 65% in der Anatas-Modifikation vorliegt.

In der in Komponente (B) vorliegenden wenigstens einen porösen Hülle liegen in einer bevorzugten Ausführungsform Metall- oder Halbmetalloxide enthaltend Elemente der Gruppen I bis XV des Periodensystems der Elemente (nach IUPAC), Lanthanoiden, Actinoiden und Mischungen davon, vor, besonders bevorzugt ausgewählt aus der Gruppe bestehend aus V, Ti, Zr, Ce, Mo, Bi, Zn, Mn, Si, Ba, Au, Ag, Pd, Pt, Ru, Rh, La und Mischungen davon. Ganz besonders bevorzugte Metall- oder Halbmetalloxide, die in der wenigstens einen porösen Hülle von Komponente (B) vorliegen, sind SiO₂, ZnO, CeO₂, TiO₂, SnO oder Mischungen davon.

In einer bevorzugten Ausführungsform des erfindungsgemäßen Beschichtungsmaterials weist Komponente (B) einen nicht porösen Kern aus TiO₂ und eine poröse Hülle aus SiO₂ auf.

Die als Komponente (B) in dem erfindungsgemäßen Beschichtungsmaterial vorliegenden photokatalytisch aktiven Teilchen weisen bevorzugt eine enge Teilchengrößenverteilung auf. Im Rahmen der vorliegenden Erfindung bedeutet eine enge Teilchengrößenverteilung, dass bevorzugt ≥ 70%, besonderes bevorzugt ≥ 80%, ganz besonders bevorzugt ≥ 90% der Teilchengrößen in einem Bereich liegen, der höchstens 20 nm, bevorzugt höchstens 15 nm, besonders bevorzugt höchstens 10 nm von der durchschnittlichen Teilchengrößen abweicht.

Die Kern-Hülle-Nanopartikel zeichnen sich dadurch aus, dass sie einen nicht porösen Kern und eine poröse Beschichtung aufweisen. Durch die gezielt einstellbare Porosität und Dicke der Beschichtung ist es möglich, die katalytische Aktivität des Kerns gezielt auf die entsprechenden Anforderungen an das erfindungsgemäße Beschichtungsmaterial einzustellen. Die Porösität der SiO₂-Schicht wird über XPS (X-ray Photo Electron Spectroscopy - ESCA Electron Spectroscopy for Chemical Analysis) gemessen. So ist es möglich, die Aktivität der Kern-Hülle-Partikel so einzustellen, dass an dem erfindungsgemäßen Beschichtungsmaterial anhaftenden Verunreinigungen katalytisch abgebaut werden, jedoch das in dem erfindungsgemäßen Beschichtungsmittel vorliegende Bindemittel im Wesentlichen nicht katalytisch abgebaut wird.

Die Photoaktivität gegen fluide Schadstoffe, beispielsweise auf dem Beschichtungsmittel anhaftenden Verunreinigungen, beträgt mehr als 60°% der Photoaktivität eines Standardphotokatalysators (Degussa P25), besonders bevorzugt mehr als 70%, ganz besonders bevorzugt mehr als 80%. Die Photoaktivität gegenüber fixierten Matrizen, d.h. dem als Komponente (A) in dem erfindungsgemäßen Beschichtungsmittel vorliegenden wenigstens einen Bindemittel, beträgt bevorzugt weniger als 50% der Photoaktivität eines Standardphotokatalysators (Degussa P25), besonders bevorzugt weniger als 40%, und ganz besonders bevorzugt weniger als 30%.

In einer bevorzugten Ausführungsform ist in dem erfindungsgemäßen Beschichtungsmaterial Komponente (B) aktiv gegenüber Verunreinigungen und nicht aktiv gegenüber Matrixmaterialien.

Der nicht poröse Kern des als Komponente (B) eingesetzten photokatalytisch aktiven Teilchens hat einen Durchmesser von 0,1 nm bis 1 µm und die Hülle des Teilchens hat eine mittlere Schichtdicke von 0,1 bis 10 nm. Der nicht poröse Kern hat dabei einen Durchmesser von mindestens 0,1 nm. In einer bevorzugten Ausführungsform beträgt der Durchmesser des nicht porösen Kerns 1 bis 200 nm, besonders bevorzugt 5 bis 50 nm, In einer weiteren bevorzugten Ausführungsform beträgt die mittlere Schichtdicke der porösen Hülle 0,1 bis 5 nm, besonders bevorzugt 1 bis 3 nm.

Die Porosität der Hülle des erfindungsgemäß als Komponente (B) eingesetzten photokatalytisch aktiven Teilchens kann ausgedrückt werden durch das Verhältnis von Anteil des Metalls in der Hülle, beispielsweise Si, in Atomprozent zu Anteil des Metalls im Kern, beispielsweise Ti, in Atomprozent und beträgt bevorzugt 2 bis 80, besonders bevorzugt 5 bis 60, insbesondere bevorzugt 8 bis 40, jeweils gemessen über XPS (X-Ray Photo Electron Spectroscopy-ESCA Electron Spectroscopy for Chemical Analysis).

Die in dem erfindungsgemäßen Beschichtungsmaterial als Komponente (B) bevorzugt eingesetzten photokatalytisch aktiven Teilchen können durch alle dem Fachmann bekannten Verfahren hergestellt werden. Beispielhaft sind als geeignete Verfahren zur Herstellung der erfindungsgemäß eingesetzten photokatalytisch aktiven Teilchen die nass-chemischen Sol-Gel-Verfahren und die Flammensynthese-Verfahren genannt. Diese Verfahren sind dem Fachmann bekannt.

### Komponente (C):

Das erfindungsgemäße Beschichtungsmaterial enthält als Komponente (C) gegebenenfalls ein Pigment.

In dem erfindungsgemäßen Beschichtungsmaterial können alle dem Fachmann bekannten und für diese Anwendung geeigneten Pigmente verwendet werden. In einer bevorzugten Ausführungsform enthält das erfindungsgemäße Beschichtungsmaterial als Komponente (C) wenigstens ein Pigment ausgewählt aus der Gruppe bestehend aus Titandioxid, Bariumsulfat, Zinkoxid, farbigen Pigmenten wie Eisenoxide, Ruß, Zinkgelb, Zinkgrün, Ultramarin, luminiszierenden oder fluoreszierenden Pigmenten, oder Azo-, Isoindolinon- und Isoindolin-, Phthalocyanin-, Quinacridon-, Perinon-, Perylen-, Anthraquinon-, Diketopyrrolopyrrol-, Thioindigo-, Dioxazin-, Triphenylmethan-, Quinophthalon-Pigmenten und Mischungen davon.

Ein geeignetes Pigment ist beispielsweise als TiO₂ in der Rutil-Modifiktion erhältlich.

Komponente (C) liegt bevorzugt in einer Menge von 5 bis 30 Gew.-%, bevorzugt 10 bis 20 Gew.-% vor.

Das erfindungsgemäße Beschichtungsmaterial enthält im Allgemeinen 30 bis 75 Gew.-% und vorzugsweise 40 bis 65 Gew.-% nichtflüchtige Bestandteile. Hierunter sind alle Bestandteile des Beschichtungsmittels zu verstehen, die nicht Wasser sind, zumindest aber die Gesamtmenge an Bindemittel (Komponente (A)), gegebenenfalls Pigment, gegebenenfalls Füllstoff (Komponente (E)) und polymere Hilfsmittel.

### Komponente (D):

Das erfindungsgemäße Beschichtungsmaterial kann als Komponente (D) gegebenenfalls ein Lösungsmittel enthalten.

Als Komponente (D) können in dem erfindungsgemäßen Beschichtungsmaterial alle dem Fachmann für die vorliegende Anwendung bekannten und geeigneten Lösungsmittel und Lösungsmittel-Gemische verwendet werden.

In einer bevorzugten Ausführungsform ist das als Komponente (D) vorliegende Lösungsmittel im Wesentlichen Wasser. Im Wesentlichen bedeutet hierbei, dass bevorzugt wenigstens 75 Gew.-% des Lösungsmittels Wasser sind, besonders bevorzugt wenigstens 90 Gew.-%.

Zur Verbesserung der Filmbildung, insbesondere bei niedrigen Temperaturen bei der Verarbeitung kann man der Farbformulierung flüchtige organische Löse- und Filmbildehilfsmittel zugeben. Beispiele für organische Löse- und Filmbildehilfsmittel sind flüchtige Kohlenwasserstoffe wie Benzinfraktionen, Weißöle, flüssige Paraffine, Glykole wie Butylenglykol, Ethylenglykol, Diethylenglykol und Propylenglykol, Glykolether wie Glykolbutylether, Diethylenglykol-monobutylether,1-Methoxy-2-propanol, Dipropylenglykolmethylether, Dipropylenglykolpropylether, Dipropylenglykol-n-buthylether, Tripropylenglykol-n-butylether, 2,3-Phenoxypropanol, Glykolester und -etherester wie Butylglykolacetat, Diethylenglykol-mono-n-butyletheracetat, 2,2,4-Trimethylpentan-1,3-diolmonoisobutyrat und vergleichbare, sowie organische Weichmacher (organische Flüssigkeiten mit einem Siedepunkt oberhalb 250°C), wie Dibutylphthalat, Dioctylphthalat, Tributoxyoctylphosphat, 2,2,4-Trimethylpentan-1,3-dioldiisobutyrat und Polypropylenglykolalkylphenylether und - herstellungsbedingt - auch nicht polymerisierte Monomere, so genannte Restmonomere. Je nach Anwendungszweck ist die Art und Menge des Filmbildehilfsmittels auszuwählen.

Komponente (D) liegt, falls vorhanden, in einer Menge von 0,1 bis 75 Gew.-% vor.

Als Komponente (E) liegt in dem erfindungsgemäßen Beschichtungsmaterial gegebenenfalls wenigstens ein Füllstoff vor. Dieser wenigstens eine Füllstoff hat im Allgemeinen in dem erfindungsgemäßen Beschichtungsmaterial die Aufgabe, die Viskosität des, bevorzugt in flüssiger Form auf das zu beschichtende Material aufzubringende Beschichtungsmaterial, zu erhöhen, um eine gute Verarbeitbarkeit zu gewährleisten.

In einer bevorzugten Ausführungsform ist der wenigstens eine Füllstoff ausgewählt aus der Gruppe bestehend aus Mineralien, beispielsweise anorganische Salze der Alkali- und Erdalkalimetalle, sowie der Übergangsmetalle und der übrigen Metalle und der Halbmetalle der Hauptgruppen des Periodensystems der Elemente, beispielsweise Mg, Ca und/oder Si. Als Anionen weisen diese Salze bevorzugt Oxid-, Carbonat-, Hydrogencarbonat-, Sulfat-, Halogenid- und stickstoffhaltige Anionen, bevorzugt Chlorid- und/oder Oxid-Anionen auf. Geeignete Füllstoffe umfassen Alumosilicate, wie Feldspäte, Silicate, wie Kaolin, Talkum, Glimmer, Wollastonit, Magnesit, Erdalkalicarbonate, wie Calciumcarbonat, beispielsweise in Form von Calcit oder Kreide, Magnesiumcarbonat, Dolomit, Erdalkalisulfate, wie Calciumsulfat, Siliciumdioxid etc. Die Füllstoffe können als Einzelkomponenten eingesetzt werden. In der Praxis haben sich Füllstoffmischungen besonders bewährt, z.B. Calciumcarbonat/Kaolin, Calciumcarbonat/Talkum. Geeignete Füllstoffe, die als Komponente (E) in dem erfindungsgemäßen Beschichtungsmaterial eingesetzt werden können, sind dem Fachmann bekannt.

Komponente (E) liegt in dem erfindungsgemäßen Beschichtungsmaterial, falls vorhanden, in einer Menge von 10 bis 40 Gew.-%, bevorzugt 15 bis 30 Gew.-%, jeweils bezogen auf das gesamte Beschichtungsmaterial, vor.

Die Pigmentvolumenkonzentration PVK des erfindungsgemäßen Beschichtungsmaterials beträgt 20 bis 65. Beispielsweise liegt die PVK in Beschichtungsmaterialien in Form von Dispersionsfarben für Außenanwendungen vorzugsweise bei 20 bis 60, insbesondere bei 30 bis 60, und speziell bei 35 bis 59.

Zur Erhöhung der Deckkraft und zur Einsparung von Weißpigmenten werden in Dispersionsfarben mit überkritischer Formulierung (hochgefüllte Farben; PVK > PVK kritisch) häufig feinteilige Füllstoffe, z.B. feinteiliges Calciumcarbonat oder Mischungen verschiedener Calciumcarbonate mit unterschiedlichen Teilchengrößen eingesetzt. Zur Einstellung der Deckkraft, des Farbtons und der Farbtiefe werden vorzugsweise Abmischungen aus Farbpigmenten und Füllstoffen eingesetzt.

Als Komponente (F) liegt in dem erfindungsgemäßen Beschichtungsmaterial gegebenenfalls wenigstens ein Pigmentverteiler vor.

Komponente (F) ist im Allgemeinen ein Homo- oder Copolymer aufgebaut aus ethylenisch ungesättigten Monomeren, die radikalisch polymerisiert werden. Beispiele für geeignete Monomere sind α,β-ungesättigte Mono- und Dicarbonsäuren, beispielsweise Acrylsäure, Methacrylsäure, Fumarsäure, Maleinsäure, Itaconsäure, und deren Mono- oder Diester mit aliphatischen oder aromatischen ein- oder mehrwertigen Alkoholen mit 1 bis 12 C-Atomen, wie Methanol, Ethanol, Propanole, wie iso-Propanol, n-Propanol, Butanole, wie n-Butanol, iso-Butanol, tert.-Butanol und Cyclohexanol. Weitere geeignete Monomere sind ethylenisch ungesättigte, aromatische Monomere wie Styrol, α-Methylstyrol. Weitere geeignete Monomere sind auch die Salze der genannten Säuren von einwertigen Kationen, beispielsweise Ammoniumacrylat.

Besonders bevorzugt werden als Komponente (F) in dem erfindungsgemäßen Beschichtungsmaterial Homo- und Copolymere auf Basis von Acrylsäureestern, beispielsweise Butylacrylat und/oder Styrol eingesetzt. Geeignete Pigmentverteiler, die als Komponente (F) in dem erfindungsgemäßen Beschichtungsmaterial eingesetzt werden können, sind dem Fachmann bekannt.

Komponente (F) liegt in dem erfindungsgemäßen Beschichtungsmaterial, falls vorhanden, in einer Menge von 0,1 bis 5 Gew.-%, bevorzugt 0,2 bis 3 Gew.-%, jeweils bezogen auf das gesamte Beschichtungsmaterial, vor.

In dem erfindungsgemäßen Beschichtungsmaterial liegt gegebenenfalls als Komponente (G) wenigstens ein Verdicker vor.

Komponente (G) ist in dem erfindungsgemäßen Beschichtungsmaterial gegebenenfalls anwesend, um die Viskosität des Materials einzustellen, und so beispielsweise die Verarbeitbarkeit zu verbessern.

Beispiele für erfindungsgemäß als Komponente (G) einsetzbare Substanzen sind beispielsweise wässrige Lösungen auf Basis Methylcellulose oder Cellulose. Diese wässrigen Lösungen weisen im Allgemeinen eine Konzentration von 0,1 bis 10 Gew.-% auf. Die Viskosität der als Verdicker eingesetzten wässrigen Lösungen beträgt im Allgemeinen bei Raumtemperatur 5000 bis 30000 mPas, beispielsweise 15000 bis 25000 mPas. Die Verdicker können auch in Substanz, d.h. ohne Lösungsmittel, in das erfindungsgemäße Beschichtungsmaterial eingebracht werden.

Wird Komponente (G) als wässrige Lösung in das erfindungsgemäße Beschichtungsmaterial eingebracht, so beträgt die Menge dieser Lösung, falls vorhanden, 2 bis 20 Gew.-%, bevorzugt 8 bis 15 Gew.-%, jeweils bezogen auf das gesamte Beschichtungsmaterial. Wird Komponente (F) in Substanz eingebracht, so geschieht dies in einer Menge von 0,1 bis 2 Gew.-%, bevorzugt 0,2 bis 1,5 Gew.-%, jeweils bezogen auf das gesamte Beschichtungsmaterial.

Als Komponenten (H) können in das erfindungsgemäße Beschichtungsmaterial ein oder mehrere Stoff(e) eingebracht werden ausgewählt aus der Gruppe bestehend aus Wasserenthärtern, Dispergierhilfsmitteln, Mitteln zur Einstellung des pH-Wertes, Emulgatoren, Konservierungsmitteln, Tensiden, Schäumern, Entschäumern, Polymeren und Mischungen davon.

Komponenten, die die genannten Aufgaben in dem erfindungsgemäßen Beschichtungsmittel erfüllen, sind dem Fachmann bekannt und kommerziell erhältlich.

Beispiele für Wasserenthärter, die gleichzeitig als Dispergierhilfsmittel fungieren sind Phosphonate und Phosphate, beispielsweise Natriumpolyphosphat.

Beispiele für Mittel zur Einstellung des pH-Wertes sind organische, wässrige oder feste Säuren und Basen, bevorzugt mittelstarke Basen, beispielsweise NH₃ in wässriger Lösung.

Als Emulgator kann man anionische, kationische oder vorzugsweise nicht-ionische oberflächenaktive Substanzen verwenden. Beispiele fur geeignete kationische Emulgatoren sind beispielsweise einen C6-C18-Alkyl-, -Aralkyl- oder heterocyclischen Rest aufweisende primäre, sekundäre, tertiäre oder quartäre Ammoniumsalze, Alkanolammoniumsalze, Pyridiniumsalze, Imidazoliniumsalze, Oxazoliniumsalze, Morpholiniumsalze, Thiazoliniumsalze sowie Salze von Aminoxiden, Chinoliniumsalze, Isochinoliniumsalze, Tropyliumsalze, Sulfoniumsalze und Phosphoniumsalze. Beispielhaft genannt seien Dodecylammoniumacetat oder das entsprechende Hydrochlorid, die Chloride oder Acetate der verschiedenen 2-(N,N,N-Trimethylammonium)ethylparaffinsäureester, N-Cetylpyridiniumchlorid, N-Laurylpyridiniumsulfat sowie N-Cetyl-N,N,N-trimethylammoniumbromid, N-Dodecyl-N,N,N-trimethylammoniumbromid, N,N-Distearyl-N,N-dimethylammoniumchlorid sowie das Gemini-Tensid N,N'-(Lauryldimethyl)ethylendiamindibromid. Beispiele fur geeignete anionische Emulgatoren sind Alkalimetall- und Ammoniumsalze von Alkylsulfaten (Alkylrest: C8 bis C12), von Schwefelsaurehalbestern ethoxylierter Alkanole (Ethoxylierungsgrad: 4 bis 30, Alkylrest: C12-C18) und ethoxylierter Alkylphenole (Ethoxylierungsgrad: 3 bis 50, Alkylrest: C4-C12), von Alkylsulfonsäuren (Alkylrest: C12-C18), von Alkylarylsulfonsäuren (Alkylrest: C9-C18) und von Sulfosuccinaten wie beispielsweise Sulfobernsteinsäuremono- oder diestern.

Tenside können in dem erfindungsgemäßen Beschichtungsmaterial eingesetzt werden, um die Verträglichkeit des Beschichtungsmaterials mit den zu beschichtenden Flächen zu erhöhen und/oder, um die Lagerstabilität zu verbessern.

Gegebenenfalls vorliegende Entschäumer werden zugesetzt, um ein Aufschäumen des erfindungsgemäßen Beschichtungsmaterials bei der Verarbeitung und/oder dem Transport zu vermeiden.

Komponente (H) liegt in dem erfindungsgemäßen Beschichtungsmaterial, falls vorhanden, insgesamt, bevorzugt in einer Menge von 1 bis 30 Gew.-%, bevorzugt 2 bis 25 Gew.-%, jeweils bezogen auf das gesamte erfindungsgemäße Beschichtungsmaterial, vor.

Die Mengen der in dem erfindungsgemäßen Beschichtungsmittel vorliegende Komponente A, B und gegebenenfalls C, D, E, F, G und/oder H addieren sich zu 100 Gew.-%.

Die vorliegende Erfindung betrifft auch ein Verfahren zur Herstellung eines erfindungsgemäßen Beschichtungsmittels, wobei die Komponenten (A) und (B) und gegebenenfalls (C), (D), (E), (F), (G) und/oder (H) vermischt werden.

Das Vermischen findet in einer bevorzugten Ausführungsform bei einer Temperatur von 5 bis 40°C, besonders bevorzugt 15 bis 35°C statt.

Erfindungsgemäß werden die photokatalytisch aktiven Teilchen (Komponente (B)) vor der Verarbeitung, d.h. der Beschichtung der zu beschichtenden Oberflächen mit dem Beschichtungsmittel, mit dem Beschichtungsmaterial vermischt, und werden nicht erst nach Aufbringen des Beschichtungsmaterials auf das zu beschichtende Material, beispielsweise durch Sprühen, auf das Beschichtungsmaterial aufgebracht.

In einer weiteren bevorzugten Ausführungsform werden alle in dem erfindungsgemäßen Beschichtungsmaterial vorliegenden Komponenten außer dem photokatalytisch aktiven Teilchen vermischt, und das wenigstens eine photokatalytisch aktive Teilchen wird als letzte Komponente in die Mischung der anderen Komponenten eingebracht.

Die vorliegende Erfindung betrifft auch die Verwendung des erfindungsgemäßen Beschichtungsmaterials zur Beschichtung von metallischen, organischen und anorganischen Materialien für Innen- und Außenanwendungen für technische Apparate.

Die vorliegende Erfindung betrifft auch die Verwendung des erfindungsgemäßen Beschichtungsmaterials zur Beschichtung von metallischen, organischen und anorganischen Materialien für Innen- und Außenanwendungen in der Bauchemie.

Die vorliegende Erfindung betrifft die Verwendung des erfindungsgemäßen Beschichtungsmaterials in Anwendungen, die Schmutzabbau-, deodorisierende-, Luftreinigende-, Wasserreinigende-, anti-bakterielle-, superhydrophile- und/oder Anti-Beschlag-Wirkungen aufweisen.

Insbesondere betrifft die vorliegende Erfindung die Verwendung des erfindungsgemäßen Beschichtungsmaterials zur Beschichtung von Materialien ausgewählt aus der Gruppe bestehend aus Baumaterialien, Verpackungsmaterialien, Wasser- und Luftreinigungseinrichtungen, Tapeten, Kunststoffe, Innen- und Außenbeleuchtungen, Vorrichtungen, Glasprodukte, Haushaltsgeräte, landwirtschaftliche Materialien/Ausrüstung, Werkzeuge, landwirtschaftliche Objekte, Tischwaren wie Geschirr und Besteck, Badprodukte, Toilettenartikel, Möbel, Filter, Maschinenabdeckungen, Fahrzeugabdeckungen, Dächer, Dachabdeckungen, Dachrinnen, Fenster- und Türrahmen, Automobilteile, Motorfahrzeuge, Schienenfahrzeuge, Flugzeuge, Raumschiffe, Behälter, Fahrräder, Motorräder, Anzeigetafeln, Verkehrsschilder, Tapete, Schallschutzwände, Treibhäuser, Isolatoren, Reflektionsplatten, Solarzellen, Solarwassererhitzer, Statuen, Zäune, Terrassen, Ventilatoren, Klimaanlagen, Bank, Rollladen, Anzeigetafel, Spiegel, Linsen, beispielsweise Brillenglas, optische Linsen, Kameralinse, Endoskop-Linse, Linse zur Herstellung von Halbleitern, Prisma, Helme, Masken, Abzeichen, Textilien (Gewebe und Vliesstoffe), Lederprodukte, Papierprodukte, Sportgeräte, gesundheitliche Instrumente, medizinische Güter, Container, Rohre, Kabel, Sanitärprodukte, u.a.

Ein spezieller Gegenstand der vorliegenden Erfindung ist die Verwendung eines erfindungsgemäßen Beschichtungsmaterials zur Beschichtung von Textilien. Unter Textilien im Sinne der vorliegenden Erfindung sind im Rahmen der vorliegenden Erfindung Textilfasern, textile Halb- und Fertigfabrikate und daraus hergestellte Fertigwaren zu verstehen, die neben Textilien für die Bekleidungsindustrie beispielsweise auch Teppiche und andere Heimtextilien sowie technischen Zwecken dienende textile Gebilde umfassen. Dazu gehören auch ungeformte Gebilde wie beispielsweise Flocken, linienförmige Gebilde wie Bindfäden, Fäden, Garne, Leinen, Schnüre, Seile, Zwirne sowie Körpergebilde wie beispielsweise Filze, Gewebe, Gewirke, Vliesstoffe (Non-wovens) und Watten. Die Textilien können natürlichen Ursprungs sein, beispielsweise Baumwolle, Seiden, Kaschmirwolle, Wolle, Leder, Filz oder Flachs, oder synthetisch, beispielsweise Polyamid, Polyester, Polyurethan, modifiziertem Polyester, Polyestermischgewebe, Polyamidmischgewebe, Polyacrylnitril, Polyolefin, Triacetat, Acetat, Polycarbonat, Polypropylen, Polyvinylchlorid oder Polyestermikrofasern. Textilien im Sinne der vorliegenden Erfindung können organische Stoffe sein oder aber anorganische Stoffe wie insbesondere Glasfasern, Kohlenstofffasern, Metallfasern und Glasfasergewebe. Textilien im Sinne der vorliegenden Erfindung können im Innen- oder Außenbereich angewendet werden. Beispiele für Textilien im Innenbereich sind Vorhänge, Möbel, Teppiche, Polstermöbel, Tischdecke, Fensterläden, Polster, Korbe, Taschen, Säcke, Posamenten, Spielzeuge, Tuche, Koffer, Wandteppiche, Sitzbezüge, Matratze, Decken, Toilettensitzbezüge, Weichbodenmatten, Bettlaken, Tapete, Läufer, Kleidung und Filter. Beispiele für Textilien im Außenbereich sind Markisen, Regenschirme, Sonnenschirme, Heissluftballone, Persenninge, Stoffverdecke, Gartenmöbel, Zelte, Banner, Fahnentuche, Kunstrasen, Seile, Netze, Sportbekleidung, Flaggen, Fahnen, Segel, Arbeitskleidung, Neoprenanzüge, Verdeckbezüge, Schilde, Abdeckhauben, Zirkuszelte, Maschinenabdeckungen, Fahrzeugabdeckungen, Fischteichabdeckungen, Schwimmbeckenabdeckungen und technische Textilien für nautische, medizinische, industrielle, aeronautische und Freizeitsanwendungen.

### Beispiele:

### Beispiel 1: Herstellung einer erfindungsgemäßen Wandfarbe I

**Tabelle 1**

| **Nr.** | **Inhaltsstoff** | **Funktion** | **Gew**. **Teile** |
|---|---|---|---|
| 1 | Wasser | Lösungsmittel | 125,0 |
| 2 | Ammoniumpolyacrylat 30% | Pigmentverteiler | 3,0 |
| 3 | Natriumpolyphosphat | Wasserenthärter, Dispergiermittel | 4,0 |
| 4 | Ammoniak (konz.) | pH-Wert-Regulierung | 2,0 |
| 5 | Wasserbasierte Kombination aus Chlormethyl-1-methylisothiazolonen und N-/ O-Formalen ("Parmetol A 26^{®}", Schülke & Mayr) | Konservierungsmittel | 3,0 |
| 6 | Methylcellulose, 20000 mPa s, 2%ige Lösung | Verdickungsmittel | 130,0 |
| 7 | Testbenzin (180-2100°C) | Lösungsmittel, Filmbildehilfsmittel | 13,0 |
| 8 | Diisobutylester eines Dicarbonsäuregemisches | Lösungsmittel, Filmbildehilfsmittel | 7,0 |
| 9 | Oleyl-E0₃₀-H | Nichtionisches Tensid | 10,0 |
| 10 | Titandioxid Rutil | Pigment | 150,0 |
| 11 | Calciumcarbonat, ca. 12 µm ("Omyacarb 5 GU^{®}", Omya) | Füllstoff, 90% CaCO₃ | 180,0 |
| 12 | Gefälltes Calciumcarbonat, ca. 200 mm Primärpartikel ("Socal P 2^{®}", Solvay) | Füllstoff, 100% CaCO₃ | 100,0 |
| 13 | Talkum | Füllstoff, 60% SiO₂, 30% MgO | 50,0 |
| 14 | Kombination von flüssigen Kohlenwasserstoffen, hydrophober Kieselsäure, synth. Copolymeren und nichtionischen Emuglatoren ("Agitan 280^{®}", Münzing) | Entschäumer | 3,0 |
| 15 | 50%ig, 100 nm ∅, ca. 1000 mPas, MFT 20°G ("Acronal 290 D^{®}", BASF AG) | Wässrige Dispersion eines Po-Wassrige Dispersion eines Polymers | 220 |
| | **Gesamt** | | **1000,0** |
| | | | |
| | Feststoffgehalt | Feste Bestandteile bezogen auf Gesamtgewicht | **60%** |
| | PVK | Verhältnis von (Volumen Pigment + Volumen Füllstoff) zu (Volumen Pigment + Volumen Füllstoff + Volumen Dispersion) | **60%** |

Tabelle 1 gibt die Zusammensetzung der Wandfarbe I wieder.

Der verwendete erfindungsgemäße Photokatalysator war TiO₂ in der Anatas-Modifikation im Kern, umgeben von porösem SiO₂ als Hülle. Der Durchmesser des Kerns betrug 5 bis 20 nm, die Dicke der Hülle betrug 1 bis 2 nm. Die Menge an Si bezogen auf das gesamte Teilchen betrug 1,1 Gew.-%. Die Porosität der Hülle betrug 11,9.

Dieser Photokatalysator wurde in die Wandfarbe bei Raumtemperatur und einer relativen Luftfeuchte von 50 bis 60% eingebracht. Diese erfindungsgemäße Wandfarbe I wurde mit einer Rakel (90 µm) auf einem Glassubstrat aufgebracht. Die Konzentration des Photokatalysators betrug 1 bis 50 g/kg, im vorliegenden Beispiel 43 g/kg. Die Schicht wurde zwischen 0,5 und 10 Stunde bei 20 - 60°C getrocknet.

Es wurden jeweils auch entsprechende Wandfarben hergestellt mit Zusatz eines Standardphotokatalysator (Degussa P25) anstelle des erfindungsgemäßen Schichtphotokatalysators.

Es wurde weiterhin auch eine Wandfarbe ohne Zusatz eines Photokatalysators hergestellt.

### Beispiel 2: Entfärbungstests

Die Photoaktivität der hergestellten Wandfarben I gegenüber dem Farbstoff der Wandfarbe wurde durch die Bestimmung der photokatalytischen Entfärbung über UV-Vis-Messungen bestimmt. Die Gesamtlaufzeit der Versuche zur Überprüfung der photokatalytischen Entfärbung der Wandfarbe unter UV-Bestrahlung lag bei 6 Monaten. Die durch das in Beispiel 1 beschriebene Verfahren hergestellten Wandfarben wurden mit einer Rakel (90 µm) auf einem Glassubstrat aufgebracht und über sechs Monate mit UV-Licht bei Raumtemperatur bestrahlt. Die UV-Intensität betrug 1,5 mW/cm².

Es wurden Blindversuche zur Entfärbung der Wandfarbe unter UV-Bestrahlung mit Zusatz eines Standardphotokatalysators (Degussa P25) anstelle des erfindungsgemäßen Schichtphotokatalysators durchgeführt.

Es wurden weiterhin Blindversuche zur Entfärbung der Wandfarbe unter UV-Bestrahlung ohne Zusatz eines Photokatalysators durchgeführt.

Die Ergebnisse sind in Tabelle 2 wiedergegeben.

**Tabelle 2**

| **Wandfarbenprobe I** | **Optische Beurteilung der Probe** |
|---|---|
| Blindprobe (Wandfarbe I ohne Katalysator) | Keine Änderung der grünen Farbe |
| Wandfarbe I mit Standardphotokatalysator Degussa P25 | Klare weiße Streifen in der grünen Farbe |
| Wandfarbe I mit erfindungsgemäßem SiO₂-beschichtetem Ti0₂-Photokatalysator | Keine Änderung der grünen Farbe |

### Beispiel 3: Herstellung einer erfindungsgemäßen Wandfarbe II

**Tabelle 3**

| **Nr.** | **Inhaltsstoff** | **Funktion** | **Menge in Gew.-Teilen** |
|---|---|---|---|
| 1 | Wasser | Lösungsmittel | 45,0 |
| 2 | Hochmolekulare Hydroxyethylcellulose (2000 mPas einer 1%igen Löaung) | Verdicker | 0,6 |
| 3 | Natronlauge 20%ig | pH-Wert-Regulierung | 0,3 |
| 4 | Copolymer aus Butylacrylat und re, gelöst in Ammoniakwasser, 30% | Acrylsäu- Pigmentverteiler | 2,4 |
| 5 | Natriumpolyphosphat | Wasserenthärter, Dispergierhilfsmittel | 0,3 |
| 6 | Wasserbasierte Kombination aus Chlormethyt-1-methylisothiazolonen und N-/ O-Formalen ("Parmetol A 26^{®}" Schülke & Mayr) | Konservierungsmittel | 0,2 |
| 7 | Mischung schäumzerstörender Polysiloxane und hydrophober Feststoffe in Polyglykol ("Byk 022^{®}", Byk Chemie) | Entschäumer | 0,3 |
| **8** | **Titandioxid Anatas, umhüllt mit SiO₂, erfindungsgemäß** | **Photokatalysator** | **12,0** |
| **9** | **Titandioxid Rutil** | **Pigment** | **10,5** |
| 10 | Sehr feinteiliges Aluminiumsilikat mit 9,5% Al als Al₂O₃ und 8% Na als Na₂O mit mittlerer Ölaufnahme und sehr hohem Weißgrad ("Sipernat P 820^{®}", Degussa) | Füllstoff, Al₂O₃ 10%, SiO₂ 80% | 6,0 |
| 11 | Talcum ("Westmin 30 E^{®}", Mondo Minerals), 12 µm | Füllstoff, SiO₂ 60%, MgO 30% | 3,0 |
| 14 | Glimmer ("Micro Mica W1^{®}", Norwegian Talc AIS), 3,4 µm | Füllstoff, Al₂O₃ 30%, SiO₂ 50%, mgO 15% | 7,5 |
| 15 | Calciumcarbonat, ca. 3 µm ("Omyacarb 2 GU^{®}", Omya) | Füllstoff, CaCO₃ 100%, kleine Teilchen | 9,8 |
| 16 | Calciumcarbonat, ca. 12 µm ("Omyacarb 5 GU^{®}", Omya) | Füllstoff, CaCO₃ 100%, mittlere Teilchen | 24,8 |
| 17 | 50%ig, 150 nm ∅, ca. 50 mPas, MFT 4°G ("Acronal DS 6255^{®}", BASF AG) | Wässrige Dispersion eines Polymers aus Acrylsäureester und Styrol | 27 |
| 18 | Mischung hydrophober Komponenten in paraffinischem Mineralöl, silikonhaltig ("Byk 038^{®}", Byk Chemie) | Entschäumer für Dispersionsfarbenonsfarben | 0,3 |
| | **Gesamt** | | **150,0** |

Tabelle 3 gibt die Inhaltsstoffe der Wandfarbe II wieder.

### Beispiel 4: Verschmutzungstests mit Wandfarbe II

Die Photoaktivität von Wandfarbe II wurde gemessen. Die Wandfarbe wurde mit einer Rakel (125 µm) auf einer Lenetafolie aufgezogen. Nach 24 h wurde eine weitere Schicht mit der gleichen Spalthöhe darüber gerackelt. Die Folie wurde 7 Tage im Klimaraum getrocknet. Die Wandfarbe wurde über 25 h unter Bestrahlung im Suntest-Gerät (Fa. Atlas, Model CPS+, Schwarztafelstandard 75°C, Lichtintensität 765 W/m², kein Filter) aktiviert. Schmutz wie Kaffee, Senf, Lippenstift und Wandkreide wurde aufgetragen und 30 Minuten einwirken gelassen. Der Schmutz wurde mit einem Tuch abgewischt und anschließend mit Wasser abgespült. Die Probe wurde 24 h bei Raumtemperatur trocknen gelassen und 250 Stunden im Suntest-Gerät bestrahlt.

Es wurden Blindversuche zum Abbau von Verschmutzungen unter UV-Bestrahlung mit Zusatz eines Standardphotokatalysator (Degussa P25) anstelle des erfindungsgemäßen Schichtphotokatalysators durchgeführt.

Es wurden weiterhin Blindversuche zum Abbau von Verschmutzungen unter UV-Bestrahlung ohne Zusatz eines Photokatalysators durchgeführt. Die Ergebnisse sind in Tabelle 4 wiedergegeben.

**Tabelle 4**

| **Wandfarbenprobe II** | **Selbstreinigende Eigenschaften (% Abbau)** | | | |
|---|---|---|---|---|
| | Senf | Kaffee | Lippenstift | Wandkreide |
| Blindprobe (Wandfarbe II ohne Katalysator) | Schmutz blieb unverändert an der Oberfläche | | | |
| Wandfarbe II met Standardphotokatalysator Degussa P25 | >60% | >60% | >20% | >10% |
| Wandfarbe II mit erfindungsgemäßem SiO₂-beschichtetem Ti0₂-Photokatalysator | >90% | >90% | >95% | >30% |

### Beispiel 5: Verkreidungstest

Die Verkreidung der Wandfarbe II wurde über die Kempf-Methode bestimmt. Zum Anquellen der Gelatineschicht wurde dünnes Photopapier mit glänzender Oberfläche 4 Minuten lang in eine Schale mit Wasser gelegt und dann mit nichtfaserndem Filtrierpaper getrocknet. Das Photopapier wurde mit der Gelatineschicht auf den zu prüfenden Anstrich gelegt. Ein Stempelgerät wurde auf das Papier aufgesetzt und der abgedeckte Probeanstrich auf einer Fläche mit 40 mm Durchmesser mit einer Kraft von 250 N beansprucht (3 x durchdrücken). Das Stempelgerät wurde dann abgenommen und das Photopapier vom Anstrich abgezogen und getrocknet. Die Proben wurden in einem Suntest Gerät (Fa. Atlas, Model CPS+, Schwarztafelstandard 75°C, Lichtintensität 765 W/m², kein Filter) über einen gesamten Zeitraum von 214 Stunden bestrahlt. Der Kreidungsgrad wurde visuell beurteilt.

Der Kreidungsgrad wurde nach der folgenden Abkreidungsskala beurteilt:

| | | |
|---|---|---|
| 0 | = | 6% Pigmentteilchen auf dem Photopapier |
| 1 | = | 17 % Pigmentteilchen auf dem Photopapier |
| 2 | = | 23 % Pigmentteilchen auf dem Photopapier |
| 3 | = | 30% Pigmentteilchen auf dem Photopapier |
| 4 | = | 54 % Pigmentteilchen auf dem Photopapier |
| 5 | = | 74 % Pigmentteilchen auf dem Photopapier. |

Es wurden Blindversuche zum Abbau der Polymere unter UV-Bestrahlung mit Zusatz eines Standardphotokatalysators (Degussa P25) anstelle des erfindungsgemäßen Schichtphotokatalysators durchgeführt.

Es wurden weiterhin Blindversuche zum Abbau von organischen Stoffen unter UV-Bestrahlung ohne Zusatz eines Photokatalysators durchgeführt. Die Ergebnisse sind in Tabelle 5 wiedergegeben.

**Tabelle 5**

| **Wandfarbeprobe II** | **Kreidungsgrad** |
|---|---|
| Blindprobe (ohne Katalysator) | 0 |
| Wandfarbe II mit Standardphotokatalysator Degussa P25 | 3 |
| Wandfarbe II mit erfindungsgemäßem SiO₂-beschichtetem TiO₂-Photokatalysator | 0 |

### Beispiel 6: Herstellung einer erfindungsgemäßen Beschichtung für Textilien I

**Tabelle 6 Herstellung von Textilbeschichtung I**

| **Nr.** | **Inhaltsstoff** | **Funktion** | **Menge in Gew.Teilen** |
|---|---|---|---|
| 1 | Wasser | Lösungsmittel | 153 |
| 2 | 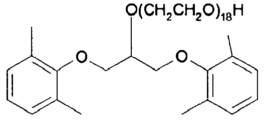 | Emulgator | 2 |
| 3 | 51 Gew. -% Lösung eines Umsetzungsprodukts von Hexamethyldiisocyanat mit n-C₁₈H₃₇(OCH₂CH₂)₁₅OH in Isopropanol/wasser (volumenanteile 2:3) | Entschäumer | 3 |
| 4 | Ammoniak (25%) | pH-Wert-Regulierung | 2 |
| 5 | Statistische Emulsionscopolymerisats von (in Gew.Teilen): Ethylacrylate 70, Butylacrylate 15, Acrylnitril 10, Acrylamid 2,6, Methylolacrylamid 1,9 sind jeweils bezogen auf gesamten Feststoff, mittlerer Partikeldurchmesser (Gewichtsmittel) 180 nm, bestimmt durch Coulter Counter, Tg = 9 °G, Molekulargewicht = 5000, Feststoffgehalt 51 % | Acrylatdispersion | 800 |
| 6 | Titandioxid | Pigment/Photokatalysator | 28 |
| 7 | 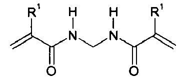 | Verdicker | 12 |
| | Synthetische Verdickungsmittel aus Copolymeren von 85 bis 95 Gew.-% Acrylsäure, 4 bis 14 Gew.-% Acrylamid und 0,01 bis maximal 1 Gew.-% des (Meth)acrylamidderivats mit Molekulargewichten im Bereich von 100.000 bis 2.000.000 g/mol, in denen die Reste R¹ gleich oder verschieden sein können und Methyl oder Wasserstoff bedeuten können. | | |
| | **Gesamt** | | **1000** |

Der verwendete erfindungsgemäße Photokatalysator war TiO₂ in der Anatas-Modifikation im Kern, umgeben von porösem SiO₂ als Hülle. Der Durchmesser des Kerns betrug 20-30 nm, die Dicke der Hülle 1 bis 2 nm. Die Menge an Si bezogen auf das gesamte Teilchen betrug 0,9 Gew.-%.

Der Photokatalysator wurde in das Beschichtungsmaterial eingebracht (gerührt über 20 Minuten bei Ultraturrax 1500 rpm) und mit einem Messer auf einem Textil (Baumwoll-Körper) aufgebracht. Die Dicke der Beschichtung betrug 0,3 mm. Die Schicht wurde 2 Minuten bei 100°C getrocknet und 2 Minuten bei 150°C fixiert.

Es wurden Blindversuche mit Zusatz von einem Standardphotokatalysator (Degussa P25) durchgeführt.

Es wurden weiterhin Blindversuche ohne Photokatalysator mit Zusatz von einem Titandioxid-Pigment in der Rutil-Modifikation durchgeführt.

### Beispiel 7: Herstellung einer erfindungsgemäßen Beschichtung für Textilien II

**Tabelle 7 Herstellung von Textilienbeschichtung II**

| **Nr.** | **Inhaltsstoff** | **Funktion** | **Menge in Gew.Teilen** |
|---|---|---|---|
| 1 | Wasser | Lösungsmittel | 128 |
| 2 | 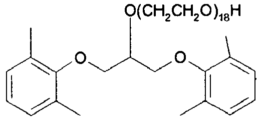 | Emulgator | 2 |
| 3 | AI(OH)₃, Al₂O₃ | Al(OH)₃, Al₂O₃ | 80 |
| 4 | Ammoniak (25%) | pH-Wert-Regulierung | 2 |
| 5 | Statistische Emulsionscopolymerisats von (in Gew.-Teilen): Ethylacrylate 70 , Butylacrylate 15, Acrylnitril 10, Acrylamid 2,6, Methylolacrylamid 1,9 sind jeweils bezogen auf gesamten Feststoff, mittlerer Partikeldurchmesser (Gewichtsmittel) 180 nm, bestimmt durch Coulter Counter, Tg = 9 °G, Molekulargewicht = 5000. Feststoffgehalt 51 Gew.-% | Acrylatdispersion | 700 |
| 6 | Titandioxid | Pigment/Photokatalysator | 40 |
| 7 | Poymer auf Basis von: | Fixierer | 5 |
| | 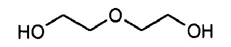 | | |
| | 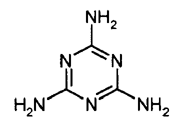 | | |
| | 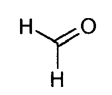 | | |
| 8 | 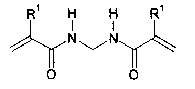 | Verdicker | 8 |
| | Synthetische Verdickungsmittel aus Copolymeren von 85 bis 95 Gew.-% Acrylsäure, 4 bis 14 Gew.-% Acrylamid und 0,01 bis maximal 1 Gew.-% des (Meth)acrylamidderivats mit Molekulargewichten im Bereich von 100.000 bis 2.000.000 g/mol, in denen die Reste R¹ gleich oder verschieden sein können und Methyl oder Wasserstoff bedeuten können. | | |
| 9 | Dinatrium-4-(octadecilaminno)-4-oxo-2-sulfonatbutyrat, wässrige Lösung, Feststoffgehalt 40 Gew.-%. | Schäumermittel | 2 |
| 10 | Ammoniumstearat | Schäumermittel | 33 |
| | **Gesamt** | | **1000** |

Der verwendete erfindungsgemäße Photokatalysator war TiO₂ in der Anatas-Modifikation im Kern, umgeben von porösem SiO₂ als Hülle. Der Durchmesser des Kerns beträgt 20-30 nm, die Dicke der Hülle 1 bis 2 nm. Die Menge an Si bezogen auf das gesamte Teilchen betrug 0,8 Gew.-%.

Der Photokatalysator wurde in das Beschichtungsmaterial eingebracht (gerührt. über 20 Minuten bei Ultraturrax 1500 rpm) und mit einem Messer auf einem Textil (Baumwoll-Körper) aufgebracht. Die Dicke der Beschichtung betrug 1,0 mm. Die Schicht wurde 3 Minuten bei 110°C und 5 Minuten bei 150°C getrocknet.

Es wurden Blindversuche mit Zusatz von einem Standardphotokatalysator (Degussa P25) durchgeführt.

Es wurden weiterhin Blindversuche ohne Photokatalysator mit Zusatz von einem Titandioxid-Pigment in der Rutil-Modifikation durchgeführt.

### Beispiel 9: Photoaktivität der beschichteten Textiloberflächen

Die Photoaktivitäten der erfindungsgemäßen Textilbeschichtungen I und II wurden über die Entfärbung von einer Methylenblau-Lösung bestimmt. 10 µL von Methylenblau-Lösungen mit Konzentrationen von 10, 25 und 100 µmol/L wurden auf die beschichtete Oberfläche des Textils aufgebracht. Das so beschichtete Textil wurde jeweils mit UV-Licht (Lichtintensität 1,5 mW/cm²) bestrahlt. Die Entfärbung des Farbstoffs Methylenblau wurde visuell über Zeit beobachtet.

Es wurden Blindversuche zur Entfärbung des Methylenblaus unter UV-Bestrahlung mit Zusatz von einem Standardphotokatalysator (Degussa P25) durchgeführt.

Es wurden weiterhin Blindversuche zur Entfärbung des Methylenblaus unter UV-Bestrahlung ohne Photokatalysator mit Zusatz von einem Titandioxid-Pigment in der Rutil-Modifikation durchgeführt.

| **Textilienprobe** | **Zeit zur vollständigen Entfärbung** | | |
|---|---|---|---|
| | 10 µmol/L | 25 mol/L | 100 µmol/L |
| | MB-Lösung | MB-Lösung | MB-Lösung |
| Blindprobe (Textilienbeschichtung II ohne Photokatalysator) | 5 Tage | 10 Tage | Nicht völlig entfärbt |
| Textilbeschichtung II mit Standardkatalysator Degussa P25 | 1 Stunde | 5 Stunden | Nicht völlig entfärbt |
| Textilbeschichtung II mit erfindungsgemäßem SiO₂-beschichtetem TiO₂-Photokatalysator | 1 Stunde | 3 Stunden | 6 Wochen |
| Textilbeschichtung I mit erfindungsgemäßem SiO₂-beschichtetem TiO₂-Photokatalysator | 7 Stunden | 3 Tage | 9 Wochen |

## Patentansprüche

1. Beschichtungsmaterial, umfassend
(A) wenigstens ein Bindemittel als Komponente (A) und
(B) wenigstens ein photokatalytisch aktives Teilchen, umfassend einen nicht porösen Kern enthaltend wenigstens ein Metall- oder Halbmetalloxid mit einem Durchmesser von 0,1 bis 1 µm und wenigstens eine den Kern zumindest teilweise umgebende poröse Hülle enthaltend wenigstens ein weiteres Metall- oder Halbmetalloxid mit einer mittleren Schichtdicke von 0,1 bis 10 nm als Komponente (B).

2. Beschichtungsmaterial nach Anspruch 1, **dadurch gekennzeichnet, dass** Komponente (A) ausgewählt ist aus der Gruppe bestehend aus auf Wasser und/oder organischen Lösungsmitteln basierenden Kunststoffdispersionen auf Basis von nicht-polymerischen Vernetzungssystemen, Acrylaten, Caprolactam, Vinylcaprolactam, N-Vinylformamid, Acrylestern, Styrol/Acrylestern und Vinylacetaten, Polyurethanen, Epoxidharzen, Alkydharzen und Mischungen davon.

3. Beschichtungsmaterial nach Anspruch 1 oder 2, **dadurch gekennzeichnet, dass** Komponenten (B) einen Kern aus TiO₂ und eine Hülle aus SiO₂ aufweist.

4. Beschichtungsmaterial nach Anspruch 3, **dadurch gekennzeichnet, dass** das TiO₂ im Wesentlichen in der Anatas-Modifikation vorliegt.

5. Beschichtungsmaterial nach einem der Ansprüche 1 bis 4, **dadurch gekennzeichnet, dass** es als Komponente (C) wenigstens ein Pigment ausgewählt aus der Gruppe bestehend aus Titandioxid, Bariumsulfat, Zinkoxid, farbigen Pigmenten, Ruß, Zinkgelb, Zinkgrün, Ultramarin, lumineszierenden oder fluoreszierenden Pigmenten, Azo-, Isoindolinon- und Isoindolin-, Phthalocyanin-, Quinacridon-, Perinon-, Perylen-, Anthraquinon-, Diketopyrrolopyrrol-, Thioindigo-, Dioxazin-, Triphenylmethan-, Quinophthalon-Pigmenten und Mischungen davon enthält.

6. Beschichtungsmaterial nach einem der Ansprüche 1 bis 5, **dadurch gekennzeichnet, dass** Komponente (B) aktiv gegenüber Verunreinigungen und nicht aktiv gegenüber Matrixmaterialien ist.

7. Verfahren zur Herstellung eines Beschichtungsmittels nach einem der Ansprüche 1 bis 6, **dadurch gekennzeichnet, dass** die Komponenten (A) und (B) vermischt werden.

8. Verwendung eines Beschichtungsmaterials nach einem der Ansprüche 1 bis 6 zur Beschichtung von metallischen, organischen und anorganischen Materialien für Innen- und Außenanwendungen in der Bauchemie.

9. Verwendung eines Beschichtungsmaterials nach einem der Ansprüche 1 bis 6 zur Beschichtung von Textilien.

10. Verwendung eines Beschichtungsmaterials nach einem der Ansprüche 1 bis 6 zur Beschichtung von metallischen, organischen und anorganischen Materialien für Innen- und Außenanwendungen für technische Apparate.

11. Verwendung eines Beschichtungsmaterials nach einem der Ansprüche 1 bis 6 in Anwendungen, die Schmutzabbau-, deodorisierende-, luftreinigende-, wasserreinigende-. anti-bakterielle-, superhydrophile- und/oder Anti-Beschlag-Wirkungen aufweisen.

## Claims

1. A coating material comprising
(A) at least one binder as component (A) and
(B) at least one photocatalytically active particle comprising a nonporous core comprising at least one metal oxide or semimetal oxide having a diameter of 0.1 to 1 µm and at least one porous envelope at least partly surrounding the core and comprising at least one further metal oxide or semimetal oxide having an average layer thickness of 0.1 to 10 nm as component (B).

2. The coating material according to claim 1 wherein component (A) is selected from the group consisting of water- and/or organic solvent-based plastics dispersions based on nonpolymeric crosslinking systems, acrylates, caprolactam, vinylcaprolactam, N-vinylformamide, acrylic esters, styrene/acrylic esters and vinyl acetates, polyurethanes, epoxy resins, alkyd resins and mixtures thereof.

3. The coating material according to claim 1 or 2 wherein component (B) has a core of TiO₂ and an envelope of SiO₂.

4. The coating material according to claim 3 wherein the TiO₂ is essentially in the anatase form.

5. The coating material according to any one of the claims 1 to 4 comprising as component (C) at least one pigment selected from the group consisting of titanium dioxide, barium sulfate, zinc oxide, colored pigments, carbon black, zinc yellow, zinc green, ultramarine, luminescent or fluorescent pigments, azo pigments, isoindolinone pigments, isoindoline pigments, phthalocyanine pigments, quinacridone pigments, perinone pigments, perylene pigments, anthraquinone pigments, diketopyrrolopyrrole pigments, thioindigo pigments, dioxazine pigments, triphenylmethane pigments, quinophthalone pigments and mixtures thereof.

6. The coating material according to any one of the claims 1 to 5 wherein component (B) is active with regard to soils and nonactive with regard to matrix materials.

7. A process for producing a coating according to any one of the claims 1 to 6, which comprises components (A) and (B) being mixed.

8. The use of a coating material according to any one of the claims 1 to 6 for coating metallic, organic and inorganic materials for interior and exterior applications in building construction chemistry.

9. The use of a coating material according to any one of the claims 1 to 6 for coating textiles.

10. The use of a coating material according to any one of the claims 1 to 6 for coating metallic, organic and inorganic materials for interior and exterior applications for technical apparatus.

11. The use of a coating material according to any one of the claims 1 to 6 in applications comprising soil-degrading, deodorizing, air-cleaning, water-cleaning, antibacterial, superhydrophilicizing and/or antifogging effects.

## Revendications

1. Matériau de revêtement, comprenant :
(A) au moins un liant en tant que composant (A) et
(B) au moins une particule photocatalytiquement active, comprenant un noyau non poreux qui contient au moins un oxyde métallique ou semi-métallique, d'un diamètre de 0,1 à 1 µm, et au moins une enveloppe poreuse entourant au moins partiellement le noyau qui contient au moins un autre oxyde métallique ou semi-métallique, d'une épaisseur de couche moyenne de 0,1 à 10 nm, en tant que composant (B).

2. Matériau de revêtement selon la revendication 1, **caractérisé en ce que** le composant (A) est choisi dans le groupe constitué par les dispersions de plastique à base d'eau et/ou de solvants organiques à base de systèmes de réticulation non polymères, d'acrylates, de caprolactame, de vinylcaprolactame, de N-vinylformamide, d'esters acryliques, d'esters styréniques/acryliques et d'acétates de vinyle, de polyuréthannes, de résines époxyde, de résines alkyde et leurs mélanges.

3. Matériau de revêtement selon la revendication 1 ou. 2, **caractérisé en ce que** le composant (B) comporte un noyau en TiO₂ et une enveloppe en SiO₂.

4. Matériau de revêtement selon la revendication 3, **caractérisé en ce que** le TiO₂ se présente essentiellement sous forme anatase.

5. Matériau de revêtement selon l'une quelconque des revendications 1 à 4, **caractérisé en ce qu'**il contient en tant que composant (C) au moins un pigment choisi dans le groupe constitué par le dioxyde de titane, le sulfate de baryum, l'oxyde de zinc, les pigments colorés, la suie, le chromate de zinc, le vert de cobalt, l'outremer, les pigments luminescents ou fluorescents, les pigments azo, isoindolinone et isoindoline, phtalocyanine, quinacridone, périnone, pérylène, anthraquinone, dicétopyrrolopyrrole, thioindigo, dioxazine, triphénylméthane, quinophtalone et leurs mélanges.

6. Matériau de revêtement selon l'une quelconque des revendications 1 à 5, **caractérisé en ce que** le composant (B) est actif contre les impuretés et non actif contre les matériaux de matrice.

7. Procédé de fabrication d'un agent de revêtement selon l'une quelconque des revendications 1 à 6, **caractérisé en ce que** les composants (A) et (B) sont mélangés.

8. Utilisation d'un matériau de revêtement selon l'une quelconque des revendications 1 à 6 pour le revêtement de matériaux métalliques, organiques et inorganiques, pour des applications intérieures et extérieures en chimie de construction.

9. Utilisation d'un matériau de revêtement selon l'une quelconque des revendications 1 à 6 pour le revêtement de textiles.

10. Utilisation d'un matériau de revêtement selon l'une quelconque des revendications 1 à 6 pour le revêtement de matériaux métalliques, organiques et inorganiques, pour des applications intérieures et extérieures pour des appareils techniques.

11. Utilisation d'un matériau de revêtement selon l'une quelconque des revendications 1 à 6 dans des applications qui comportent des effets de décomposition des salissures, de désodorisation, de purification de l'air, de purification de l'eau, antibactériens, superhydrophiles et/ou antibuée.
